# EUROPEAN PATENT SPECIFICATION

(11) **EP 1 773 206 B1**
(45) Date of publication and mention of the grant of the patent: **17.09.2008**
(21) Application number: 05756313.2
(22) Date of filing: 25.05.2005
(51) Int. Cl.: A61B 17/04, A61B 17/064

(54) **SOFT TISSUE FASTENER HAVING INTEGRAL BIASING SECTION**
WEICHTEILBEFESTIGUNG MIT INTEGRALEM VORSPANNABSCHNITT
DISPOSITIF DE FIXATION POUR TISSUS MOUS AVEC PARTIE INTEGREE DE SOLLICITATION

(30) Priority: 25.06.2004 US 876991; 06.10.2004 US 959787
(43) Date of publication of application: 18.04.2007
(73) Proprietor: ETHICON, INC., Somerville, New Jersey 08876-0151 (US)
(72) Inventor: KAMMERER, Gene W., East Brunswick, New Jersey 08816 (US); DION, Dorothy, West Orange, New Jersey 07052 (US); CIARROCCA, Scott, Stockton, New Jersey 08559 (US)
(74) Representative: Fisher, Adrian John
(86) International application number: PCT/US2005/018378
(87) International publication number: WO 2006/007190

(56) References cited:
- WO-A-03/086205
- US-A- 2 817 339
- US-A1- 2001 010 005
- US-A1- 2001 021 855

## Description

### Field of the Invention

This invention relates to surgical fasteners for soft tissue approximation and tissue repair. More particularly, it relates to fasteners for attaching tissues to each other, and attaching surgical meshes or fabrics to tissue within a deep cavity of the body, such as the pelvic cavity.

### Background of the Invention

In many situations, one piece of tissue must be attached to another piece of tissue for wound closure or the surgical repair of tissue defects. For example, an open wound or surgical incision may need to be closed following a surgical procedure, an injury may cause one piece of tissue (e.g., a tendon or pelvic floor tissue after childbirth) to become detached from another piece of tissue (e.g., a bone or pelvic muscle), or a piece of tissue may simply tear (e.g., a piece of meniscal cartilage or pelvic floor tissue).

The traditional technique for attaching one piece of soft tissue to another piece of soft tissue has involved stitching the two pieces of tissue together using sutures. However, in many circumstances such stitching is challenging either because of the time required to do it or the difficulty of stitching in a particular area of the body. Other techniques have involved using both suture and mesh to support the tissue that has been torn or become degenerated.

More recently, different types of surgical fasteners have been developed for holding together two pieces of tissue. Among the fasteners which have been developed to date are the so-called T-type fasteners, in which a rod-like head is perpendicularly mounted to the end of a length of flexible filament. Another of these fasteners is the so-called H-type fastener, in which rod-like heads are perpendicularly mounted to the two opposite ends of an intermediate, bridging flexible filament.

Examples of T-type and H-type fasteners, and their associated applicators, are disclosed in U.S. Patents Nos. 4,006,747 (Kronenthal et al.); 4,235,238 (Ogiu et al.); 4,669,474 (Richards et al.); 4,705,040 (Mueller et al.); 5,941,439 (Kammerer et al.); 6,152,935 (Kammerer et al.) and 6,156,044 (Kammerer et al.).

The H-type fasteners may have advantages over the conventional fasteners in certain minimally invasive techniques. In particular, it is known to use H-type fasteners in arthroscopic techniques such as meniscal repair as disclosed in U.S. Patent No. 5,320,633. Damage to the meniscus, such as rips or tears, has been found to be repairable if the torn pieces of the meniscus are approximated.

The H-type fasteners are believed to be effective in soft tissue repair since they are relatively easy to insert using a conventional apparatus having a cannulated distal needle wherein the needle has a longitudinal slot. One leg or tissue anchor of the H-type fastener is loaded into the cannulated needle, preferably having a slot. The needle is inserted through both sides of the soft tissue and one leg or anchor is expelled from the needle on one side of the tissue. The needle is then removed from the tissue and the other opposed leg or anchor remains in place positioned on the opposite side, thereby approximating the two sections of tissue. Another use of the H-type fastener is to attach a fabric or mesh to a surface of soft tissue. This is accomplished in the same manner as described above for the soft tissue to soft tissue approximation, except that a fabric is substituted for the first layer of tissue.

There are certain disadvantages associated with the use of existing H-type fasteners to approximate soft tissue to soft tissue across a tear or incision. There are also certain disadvantages associated with the use of existing H-type fasteners to approximate a fabric to soft tissue. One disadvantage is that the surgeon must precisely measure the distance between the edges of the tear or incision prior to inserting the H-type fastener in order to select the proper size H-type fastener. A precise measurement is necessary because the fastener must be sized to approximate and hold the opposing sides or surfaces of the tear against each other. One skilled in the art will appreciate the difficulties involved in attempting to obtain such measurements during a minimally invasive procedure in which a scope is used, or visualization is impaired due to the narrow confines within certain body cavities. The positioning and maneuvering of the applicator to deliver the fastener is also critical and difficult. Therefore the precision of the placement of the device is often not good. Consequently, the tear is often under-approximated with inadequate face-to-face contact, or over-approximated with more than adequate face-to-face contact. Furthermore, in the approximation of attaching a fabric to soft tissue the same issue is present, with the added concern that the proximal end of the fastener is large enough to secure the fabric to the tissue without the end pulling through the fabric, especially if the fabric has large pores and is flexible and extensible. In this case it is very likely that the proximal anchor end of the fastener would slip though the fabric pore when tension or stress is applied to the fabric or tissue. The same problem can occur when attempting to approximate two layers of tissue. If the tissue is soft and not dense, then the end anchor may pull through the tissue and the approximation can be lost.

Some H-type fasteners such as those shown in U.S. Patent Nos. 6,152,935 and 6,156,044, incorporate a biasing mechanism between the anchors that may be resiliently biased to bring the opposing tissue anchors closer together after insertion. The anchors,however, are designed for insertion on opposite sides of tissue to be approximated, and thus still suffer from many of the drawbacks described above.

What is needed in this art are improved surgical fasteners for approximating tissue of approximating a fabric to tissue, which overcome both the disadvantages associated with the H-type fasteners and arrow type fasteners of the prior art.

US2001/0021855A discloses a fastener having a first and second anchor in a first plane, said anchors being joined by a flexible suture, wherein the anchors are biased such that the flexible suture extends between said first and second anchors perpendicular to the plane of said first and second anchors.

### Summary of the Invention

The present invention provides a fastener as defined in claim 1 for securing a target tissue to a second element which includes a first anchor and a second anchor lying in substantially the same plane, and a connector. The connector includes a first central biasing section having a first end coupled to the first anchor and a second end, a second central biasing section having a first end coupled to the second anchor and a second end, and a connector section extending between the second ends of the first and second central biasing sections. The first and second central biasing sections are capable of being elastically deformed from a first relaxed resting position wherein the connector section is positioned a first distance from the first plane to a second extended position wherein the connector section is positioned a second distance from the first plane that is greater than the first distance and is biased toward the first plane.

The first and second anchors have first and second longitudinal axes respectively that are substantially parallel to and spaced apart from one another when the first and second biasing sections are in the resting position.

A method is also disclosed for approximating first and second tissue sections. The method includes the steps of providing a fastener having first and second central biasing sections each having first and second ends. The central biasing sections are capable of being elastically deformed from a first relaxed resting position to a second extended position. A first anchor its mounted to the first end of the first biasing section, a second anchor is mounted to the first end of the second biasing section, and a connector section extends between the second end of the first biasing section and the second end of the second biasing section. The method further includes the step of inserting the first and second anchors through the first tissue section and into the second tissue section, deforming the central biasing sections from their first relaxed resting positions to their second extended positions, positioning the connector section at the second tissue section while the central biasing sections are in their second extended positions, and releasing the central biasing sections from their second extended positions to their first relaxed positions so as to apply a biasing force between the first and second tissue sections.

Another method is disclosed for coupling a fabric to tissue. The method includes the steps of providing a fastener having first and second central biasing sections each having first and second ends. The central biasing sections are capable of being elastically deformed from a first relaxed resting position to a second extended position. A first anchor is mounted to the first end of the first biasing section, a second anchor is mounted to the first end of the second biasing section, and a connector section is connected between the second end of the first biasing section and the second end of the second biasing section, the method further includes the steps of inserting the first and second anchors through the surgical fabric and into the tissue, deforming the central biasing sections from their first relaxed resting positions to their second extended positions, positioning the connector section at but not through the fabric while the central biasing sections are in their second extended positions, and releasing the central biasing sections from their second extended positions to their first relaxed positions so as to apply a biasing force between the connector section and the first and second anchors to thereby bias the fabric toward the tissue.

The first and second biasing sections preferably lie in second and third planes both of which are substantially perpendicular to the first plane.

The features of the present invention will be more fully disclosed by the following detailed description of the preferred embodiments of the invention, which is to be considered together with the accompanying drawings wherein like numbers refer to like parts.

### Brief Description of the Drawings

FIG. 1 is a perspective view of a preferred fastener of the present invention.
FIG. 1a illustrates the fastener of Fig. 1 in an extended position.
FIG. 2 is a perspective view of an alternate embodiment of the fastener of the present invention.
FIG. 3 is a perspective view of an alternative embodiment of a fastener with pores or holes in the connector section.
FIG. 4 is a perspective view of an alternative embodiment of a fastener with an enlargement of the connector section that optionally contains a hole for suture passage.
FIG. 5 is a side view of a fastener of the present invention having biasing sections with saw-tooth configurations.
FIG. 6 is a side view of a fastener of the present invention having biasing sections with sinusoidal configurations.
FIG. 7 is a side view of a fastener of the present invention with the transition section of the central biasing section having a sloped configuration.
FIG. 8 is illustrates a fastener of the present invention with a fabric or mesh connector section.
FIG. 9 illustrates a fastener of the present invention with a suture as the connector section.
FIGS. 10, 10a, and 10b illustrate a fastener of the present invention with the biasing sections and connector section having a circular cross section.
FIG. 11 illustrates an alternative cross-sectional configurations for a fastener according to the present invention.
FIG. 12 is an illustration of a fastener loaded into an applicator.
FIGS. 13-15 illustrate various steps during deployment of a fastener using the applicator of FIG. 12.
FIG. 16 is a graph illustrating the biasing force versus displacement for a fastener of the present invention.
Fig. 17 is a cross-sectional view of the attachment of a fabric to a soft tissue utilizing a fastener of the present invention.
FIG. 18 is a top view of the attachment of a fabric to a soft tissue utilizing the fastener of FIG. 17.
FIG. 19 is a cross sectional view of the approximation of two sections of soft tissue utilizing fasteners of the present invention.
FIG. 20 illustrates suspension of the apical portion of a vagina from a soft tissue utilizing fasteners of the present invention.
FIG. 21 illustrates soft tissue to soft tissue attachment utilizing fasteners of the present invention.
FIG. 22 illustrates the attachment of a surgical fabric to soft tissue utilizing fasteners of the present invention.
FIG. 23 illustrates use of fasteners of the present invention in repairing a tear to the supraspinatus tendon of the shoulder.
FIG. 24 illustrates use of fasteners of the present invention in repairing a tear in the peroneus longus tendon in the ankle.
FIG. 25 illustrates the use of a fastener in an artery to secure a stent.

### Detailed Description of the Preferred Embodiments

The fasteners of the present invention may be made from numerous conventional biocompatible polymers, including non-absorbable, absorbable or resorbable. Suitable non-absorbable materials include polypropylene, nylon, polyethylene, polyester polyolefin and the like and equivalents thereof, and suitable absorbable and resorbable materials include polydioxanone, polygalactic acid, polylactic acid, polycaprolactone, copolymers and blends thereof as well as equivalents thereof.

Although not particularly preferred, other materials that could be used include metals such as stainless steel biasing steel and nickel-titanium alloys, ceramics, composites, and the like. Conventional manufacturing processes may also be used to form the fasteners such as injection molding, insert molding, extrusion molding, thermal bonding, solvent bonding, annealing, heat treatment, mechanical deformation, heat fusion, welding, machining cutting, or other methods known to those skilled in the art.

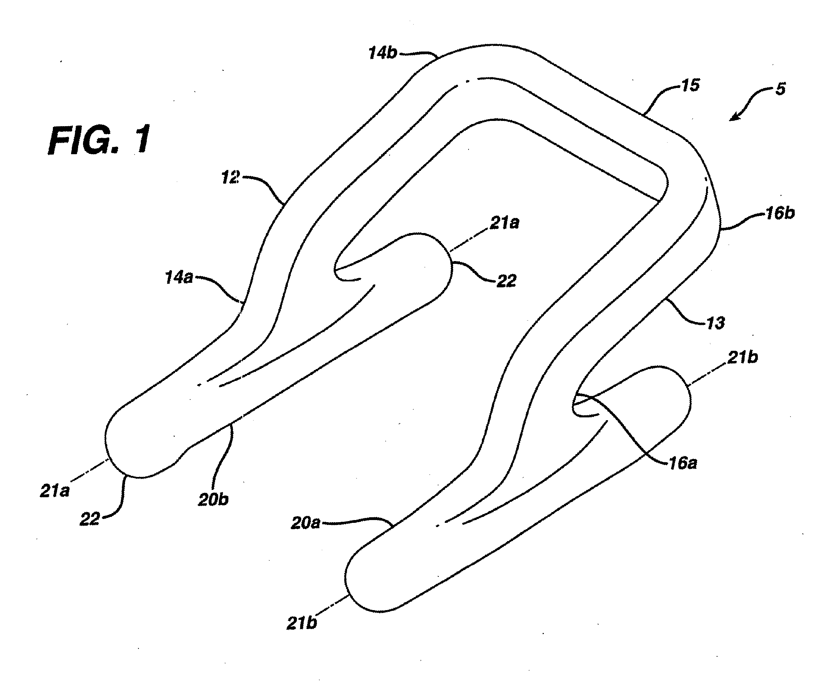

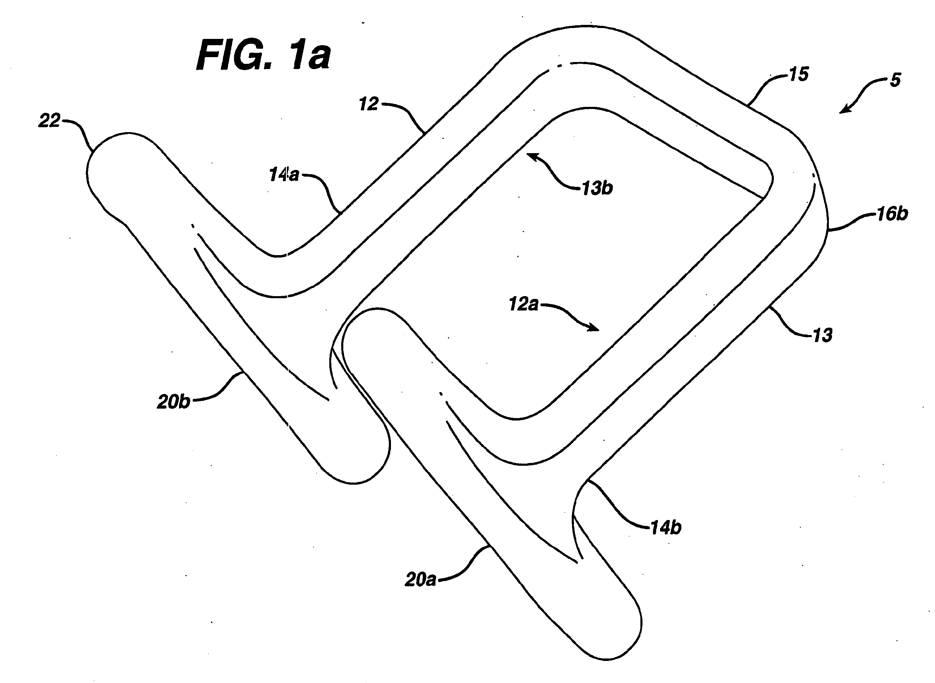

Referring now to FIG. 1, one embodiment of a fastener 10 includes a connector 5 including spaced-apart central biasing sections 12 and 13 having first and second ends, and a connector section 15 that is connected between the second ends 14b, 16b of the first and second biasing sections. Mounted to the first ends 14a, 16a of the biasing sections are tissue anchors 20a and 20b. These tissue anchors are preferably substantially rod-shaped cylinders having opposed rounded ends 22, although they may have other configurations, such as spheres, discs, beams, or the like. The ends 22 of anchors may also be pointed, flat, conical, pyramidal, or other configurations known to those skilled in the art. The tissue anchors 20a and 20b are seen to have longitudinal axes 21a and 21b, and are preferably mounted to the first ends 14a and 16a of the biasing sections 12, 13 such that the central biasing sections extend outward from the anchors in a substantially perpendicular direction at the point of connection, but then proceed to follow the curved configuration illustrated in Fig. 1. The anchors are also preferably substantially centrally mounted to the first ends 14a, 16a. The axes 21a and 21b are preferably substantially parallel to and spaced apart from one another, but may also be angulated or rotated with respect to each other. The central biasing sections 12, 13 of the fastener are elastically deformable from the relaxed position shown in Fig. 1, to the extended position shown in Fig. 1a. When in the extended position, the central biasing sections are biased to return to the shape shown in Fig. 1. Thus, when moved to the extended position during insertion, the fastener will tend to "sandwich" tissue or other elements (i.e., mesh) between the connector and the anchors 20a, 20b. This procedure will be described in greater detail below.

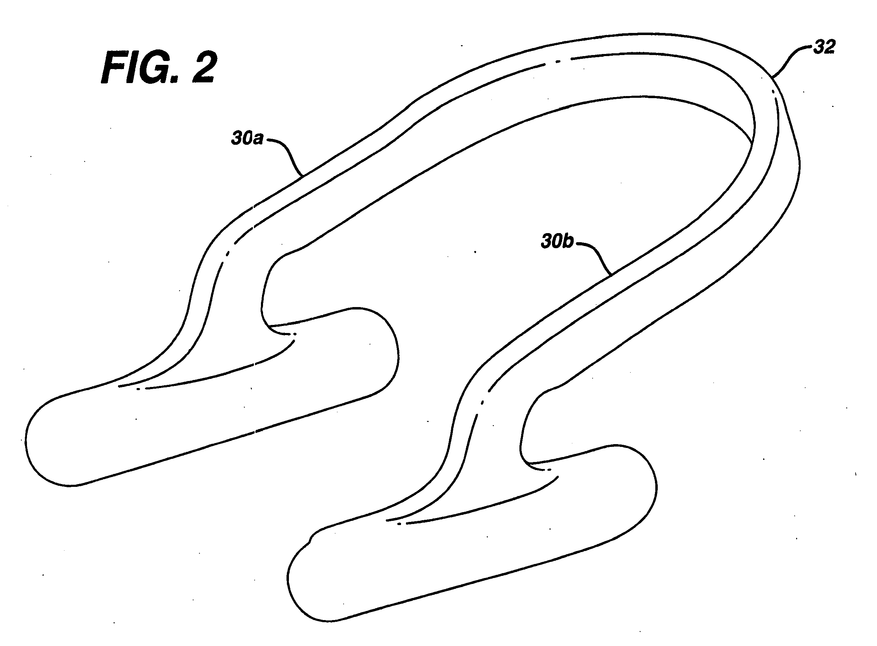

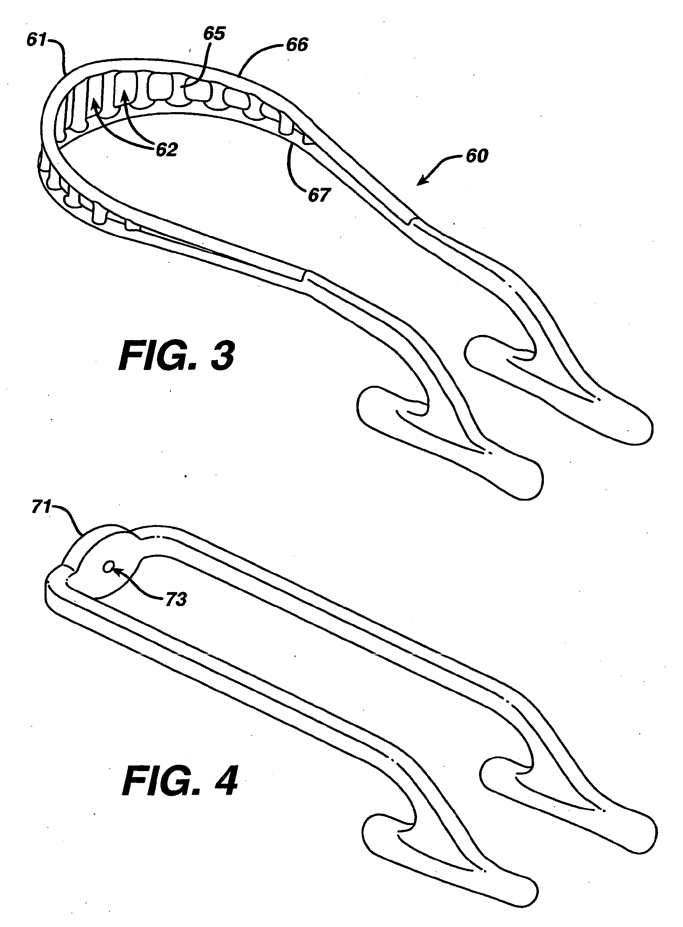

Figure 2 illustrates another embodiment of the present invention which differs from that shown in Fig. 1 in that connector section 32 has a semi-circular configuration with an approximately 180 degrees arc, although arcs of lesser magnitude may be used. It is to be understood that other aspects of the configuration of the fasteners illustrated herein may be altered without departing from the scope of the invention. For example, certain applications may render it suitable for the substantially straight portions 30a, 30b of the connector to be longer or shorter than that shown in Fig. 2. In yet another embodiment of the present invention shown in FIG.3, the connector section 61 has holes or pores 62 between solid ribs 65 that connect the top 66 of the section and the bottom 67. The illustrated openings 62 are rectangular, but can be of a different configuration, such as round, elliptical or irregular in shape. In yet another embodiment shown in Fig. 4, the connector section can simply include an enlargement area 71 that can optionally include a hole 73 for suture insertion and passage.

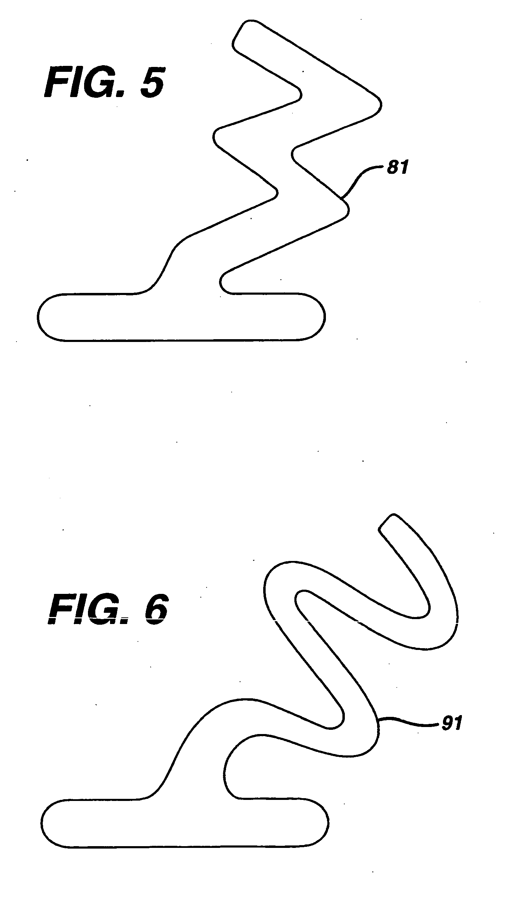

The connector of the present invention may also take on various other configurations, such as those illustrated in Figs. 5-7. Fig. 5 illustrates a fastener in which the biasing section 81 has a saw-tooth configuration, whereas Fig. 6 illustrates a fastener in which the biasing section 91 has a sinusoidal configuration.

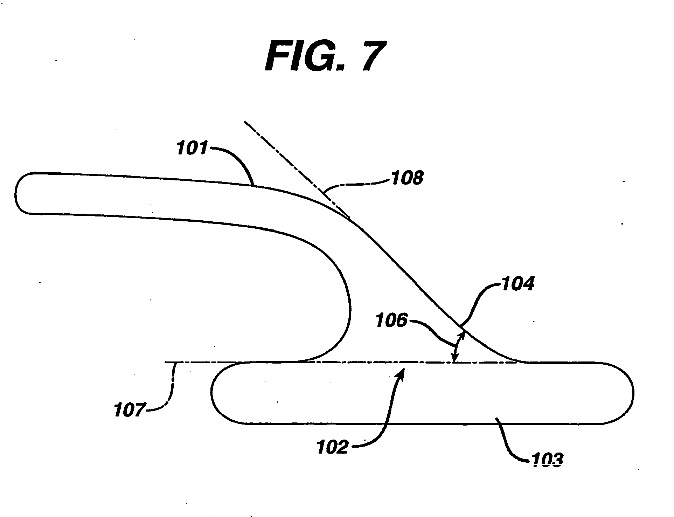

In the embodiment illustrated in FIG. 7, the biasing sections 101 connect to the mid portion 102 of the anchors 103, and include a leading edge 104. This leading edge 104 has an angular relationship with the anchors 103 as shown by lines 107 and 108, which preferably forms an angle x of approximately 45°.

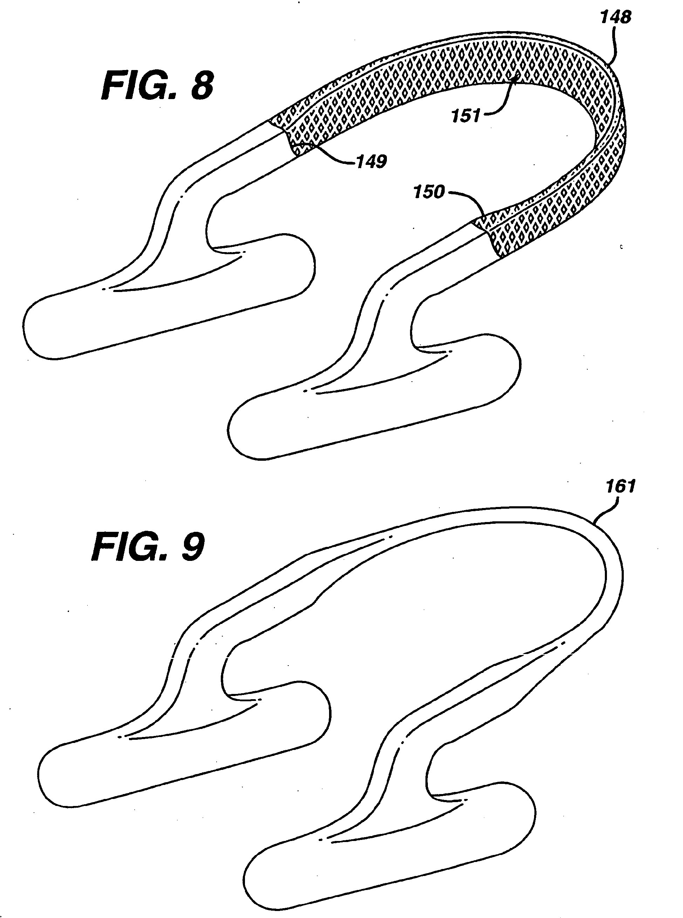

The fastener of the present invention may also include a fabric or mesh structure in the position of the connector section 148 as shown in Fig. 8. The mesh or fabric may be constructed from a biocompatible material that contains pores or holes sized to allow for tissue in growth. The coupling of the mesh or fabric connection section to the biasing sections may be mechanical, such as by crimping the ends of the biasing sections around the fabric ends 149 and 150. Alternately, the ends may be joined using a glue, such as epoxy or cyanoacrylate, may be ultrasonically welded, or melted together using other forms of energy. They can also be pinned or stapled with metal rods pushed through both ends, or alternatively sewn with a thread or wire. The fabric of the connector section 148 can contain pores 151 throughout, can be in the shape of a tape as shown or could alternately have a round cross section. In another embodiment the fabric could yet be constructed with multiple layers or could be hollow. In all cases the pore sizes of the fabric are preferably between 50 um and approximately 500 um. The fabric can be constructed using a knitting technique, a weaving techniques, a non-woven technique or a braiding technique or any combination thereof. The elements of the fabric can be made from synthetic polymers such as nylon, polypropylene, polyethylene or any other known biocompatible polymer, or could be constructed from natural materials such as cotton or rayon. Both absorbable or non-absorbable materials may be suitable as well. Some examples of absorbable materials are polycaprolactones, polyglycolic acid, polydioxanones or blends of these and similar polymers.

In yet another embodiment of the fastener of the present invention shownin FIG. 9, fastener 160 has a suture as the connector section 161. The suture 161 can be made from any suitable suture material such as nylon, polypropylene polyester and the like. Alternately the suture 161 can be made from an absorbable type material commonly used for suture, such as polydioxanone, polygalactin, polylactic acid, polygalactic acid and blends thereof.

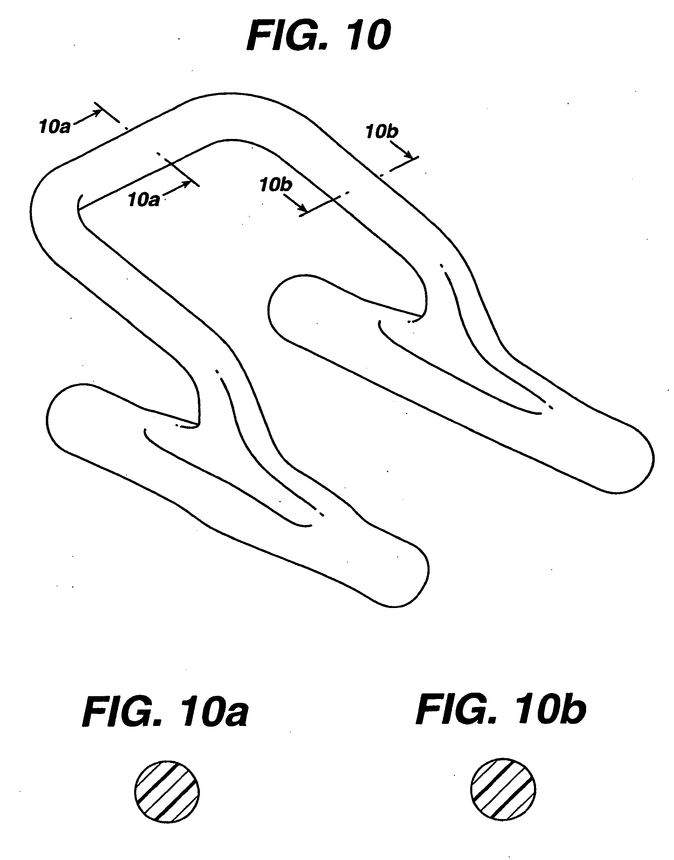

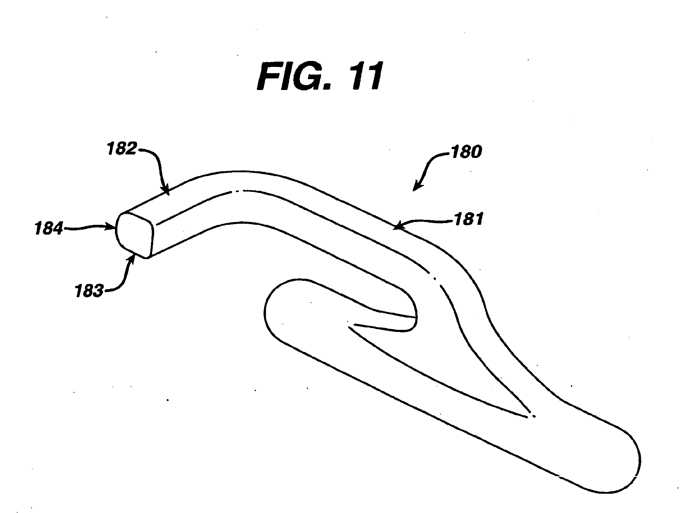

FIG. 10 illustrates a fastener of the present invention wherein the cross section of the connector section (across line a-a) and the cross section of the central biasing sections (i.e., across line b-b) are circular. These cross-sections, however, may take on various other configurations, including, but not limited to, that shown in Fig. 11, or simply a substantially rectangular cross-section. The "D" shaped cross section shown in Fig. 11 is utilized when the fasteners are manufactured by injection molding or other molding processes, which use multiple part rigid molds. The advantage of the "D" cross section is the availability of a flat surface 183, which provides a configuration for two sections of the mold to come together and for good alignment to occur there.

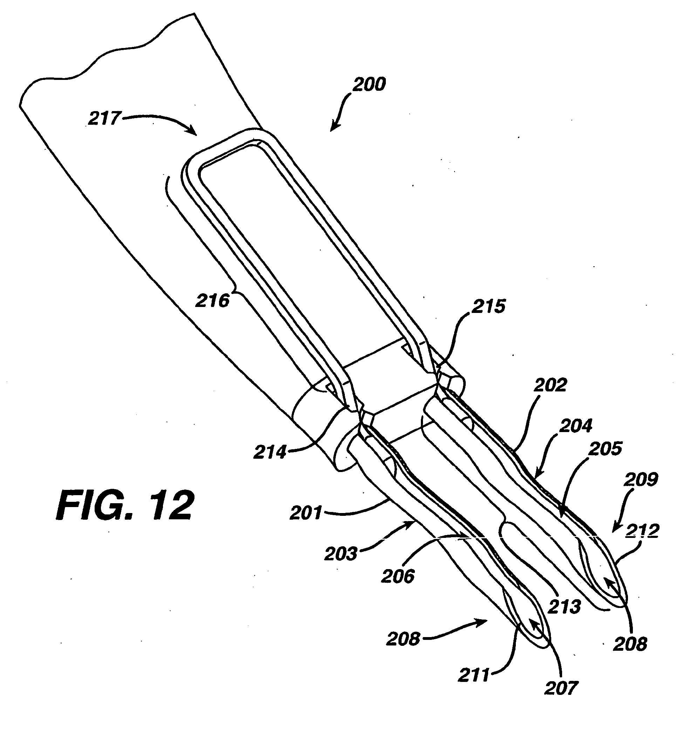

One embodiment of an applicator that can be used for implanting the fasteners described above is shown in Figs. 12-14, and is described in greater detail in published U.S. Patent Application No. 2005/0288689, which was filed on June 25, 2004. The end of the applicator 200 contains two hollow needles 201 and 202. Along the length of the top of the needles, are slots 205 and 206, which communicate with the internal lumens 207 and 208 of the needles 201 and 202. The distal ends 209 and 210 of these needles are configured with beveled edges 211 and 212 designed to ease the penetration of the needles through tissue. Placed at a specific distance 213 from the distal ends 209 and 210 are two fastener holding receptacles 214 and 215. The relationship between distance 213 and the length of the central biasing section 216 of the fastener 217 is set such that distance 213 is the same value as length of the central biasing section 216. As is seen, the legs of the fastener are inserted into the lumens 207, 208 of the needles, with the connector extending to the outside of the applicator.

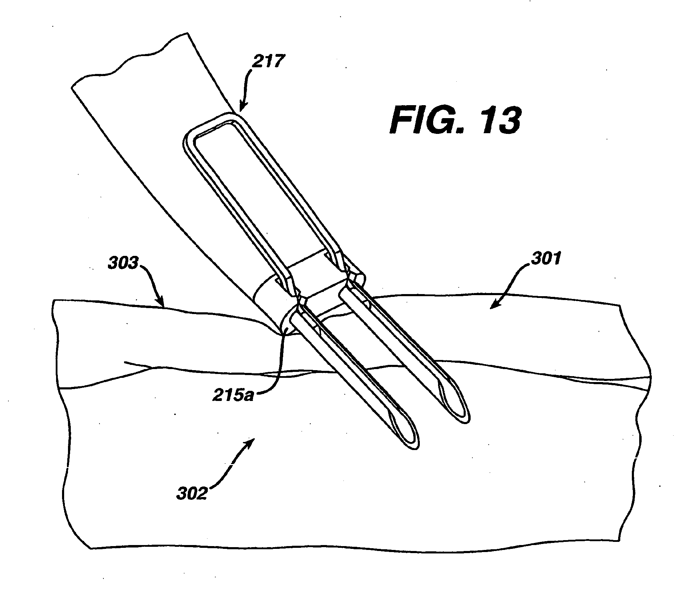

FIG 13 illustrates the position of needles 201 and 202 as they have been inserted through a top layer of tissue 301 and bottom layer of tissue 302. The fastener receptacle face 215a of the applicator 200 is in contact with the outer surface 303 of the top tissue 301. This limits the depth of penetration of the distal portion of the needles 201 and 202. The needles 201 and 202 are at an angle with the surface 303 of the top tissue 301 and with the linear plane of both the top tissue 301 and the bottom tissue 302. This angle of insertion can encompass a wide range from approximately 20° to 160° but is not limited thereto.

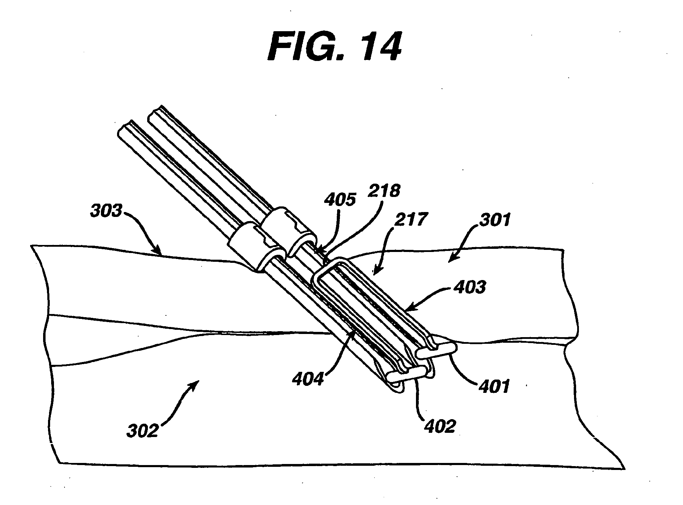

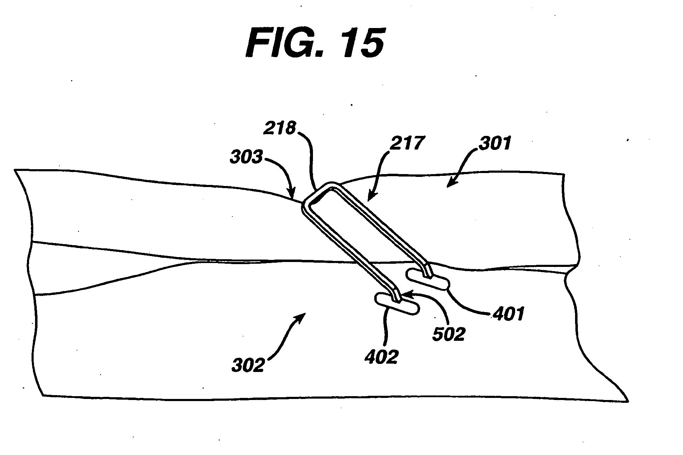

FIG. 14 illustrates the deployment of the fastener 217 within the tissues 301 and 302. The tissue anchors 401 and 402 have been pushed out of the needles 201 and 202 by a push rod or the like that extends within lumens 207, 208 so that the central biasing sections 403, 404 are substantially in the extended position as shown. The connector section 218 of fastener 217 is in contact with the surface 303 of the top tissue 301. As the central biasing sections have been elastically deformed by the applicator to the substantially extended configuration shown, the fastener is biased to return to its initial shape, which is what occurs when the fastener is released from the applicator as shown in Fig. 15. The force exerted by the biasing sections 403 and 404 in opposition to the resistance of the connector section 218 being compressed against the top tissue surface 303 causes tissue 301 and 302 to be drawn together and subsequently approximated based upon the insertion depth of the fastener 217.

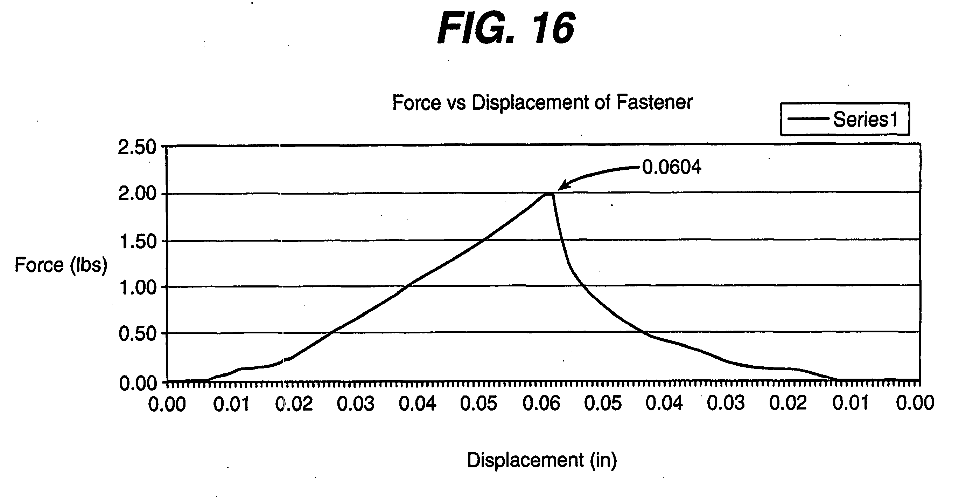

Figs. 16 is a graph illustrating the relationship between the distance the fastener is extended and the force generated through the extension and relaxation of the device. Fig. 16 shows a curve of the displacement versus force of the fastener. More particularly, it illustrates the distance required to fully extend the fastener to 1.989 lbs., maximum deflection or extension which the device undergoes during insertion, and the recovery of the fastener back to 0 lbs. force. The difference in the recovery distance is 19.8% hysteresis. The value of this testing and the graph is an example of how the transverse legs of the fastener act as biasing sections. The recovery force, although not completely 100% is enough to provide a compression force across the two layers of material being approximated, either one tissue and one fabric or two tissues. The recovery of the fastener and retention of the biasing force separate this fastener from other similar type fasteners. Additionally, due to the retention of this force through the transition of the transverse legs extension, the placement of the fastener does not have to be precise to achieve approximation. As the legs recover from the extended position to the resting position, they pull the layers of material together. Placing multiple fasteners along a line of approximation multiplies the force of compression along that line.

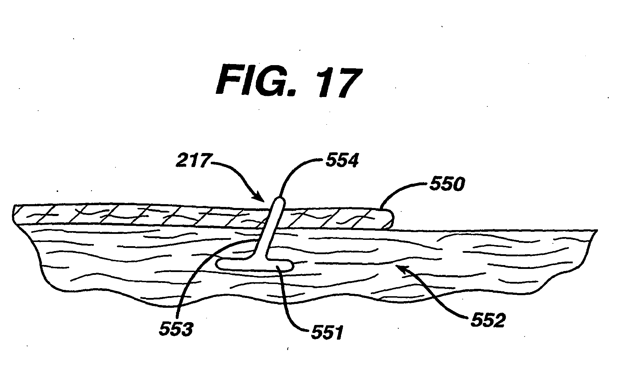

FIG. 17 is a cross-sectional view illustrating how the fastener 217 of the present invention is placed through a surgical fabric 550 with the tissue anchors 551 deployed into the soft tissue 552. The biasing section 553 is in a partially extended position and is exerting a compressive force between itself and the connector section 554 as it attempts to recover from the deformation of the deployment.

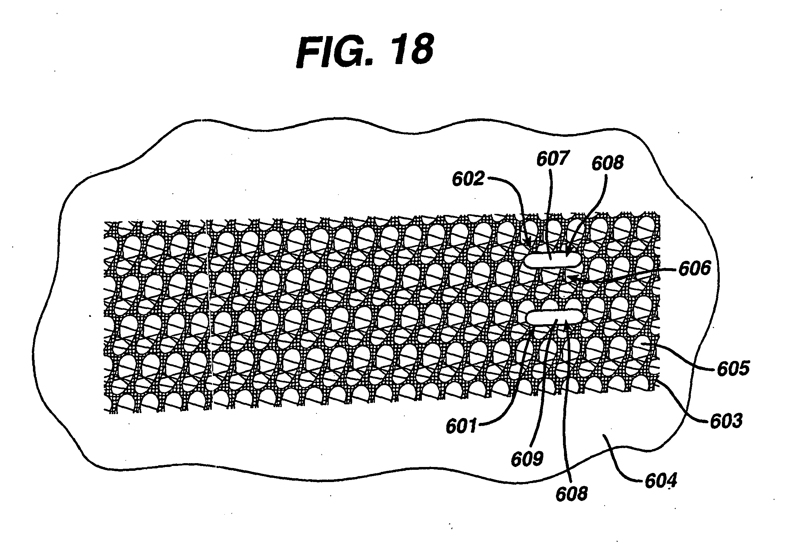

FIG. 18 is a top view of two fasteners 608, 609 that have been deployed through a fabric 603, which is a surgical mesh, and are anchored within a soft tissue 604 lying underneath the fabric. The fabric 603 contains pores 605 which are spaced across the flat surface. The two biasing sections of the fasteners (not shown) have been passed through the pores with the anchors (not shown) embedded in the tissue 604 below, and connector sections 607, 609 have captured a number of yarns 606 between the biasing sections of each fastener.

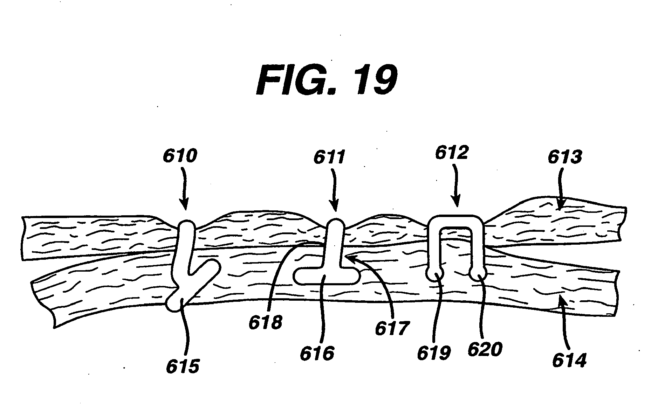

FIG. 19 illustrates a number of fasteners 610, 611 and 612, which are deployed into two layers of tissue. A top layer 613 and a bottom layer 614. The illustration shows the various deployment positions of the fasteners. Fastener 610 has its tissue anchor 615 in a partially extended position. Fastener 611 has its tissue anchor 616 in a substantially perpendicular orientation to the central biasing section 618, and thus is in a substantially fully extended position. Fastener 612 is similar to fastener 611, but is rotated 90 degrees to clearly illustrate the two anchoring points of the two anchors 619, 620. As can be further seen, inclusion of the connector section that connects between the two biasing sections bridges across the tissue or fabric between the two biasing sections, thereby reducing the criticalness of the alignment of the fastener with any tissue fiber orientation.

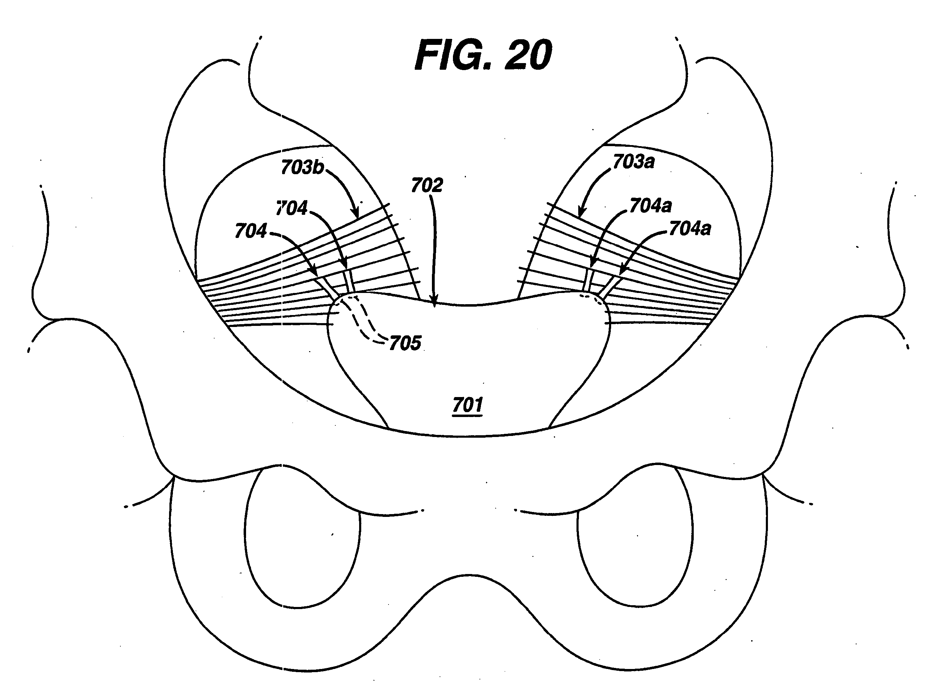

FIG. 20 illustrates a repair to a vaginal prolapse utilizing a fastener of the present invention. In this type repair the vagina 701 has prolapsed from its normal anatomical position. The apex 702 of the vagina 701 had stretched away from its attachments to the sacrospinuos ligaments 703a and 703b. To make this repair and bring the vaginal apex 702 back to a position similar to its original anatomical attachment, fasteners 704 have been placed through the tissue of the vaginal apex 702 and anchored into the tissue of the sacrospinous ligaments 703a and 703b. The fasteners 704 are shown as being placed through the tissue of the vaginal apex 702 in two different ways. The first technique shows the connector sections 705 as being outside the lumen of the vagina 701. This technique would be used in either an open or laparoscopic procedure were the surgeon would have access to the surgical site from outside the vagina 701. In the second technique, the connector sections 705a (shown in dotted lines) of the fasteners 704a are inside the lumen of the vagina. This technique would be used in a transvaginal procedure were the surgeon would have access to the surgical site through the vaginal opening. In either procedure the same type repair can be affected utilizing the fastener of the present invention. Further, when a fastener having longer central biasing sections is used, then the tissue of the vaginal apex 702 can be suspended away from the sacrospinous ligaments 703a and 703b. The value of this type suspension is that it puts the vagina 701 in a more normal anatomical position. In another embodiment of the present invention the anchor section of the fastener can be constructed from a porous fabric as illustrated in FIG. 8. Fastener 140 has a section 148 which can be made from a mesh that has openings to allow for tissue in growth. When this type fastener is utilized to suspend an organ, like the vagina, from the ligaments or other soft tissue in the pelvic cavity, and the material of the anchor section is within the vaginal tissue and also within the ligamentous tissue, then it is possible for tissue to grow from the vagina to the ligaments. This condition produces a bridge between the two, which closely simulates the appearance and function of the uteral-sacro ligaments. In most surgical cases of hysterectomy these ligaments are cut and the apex of the vagina is left without support. The use of the presently described fastener to reconnect the vagina to the sacrospinuos ligaments, where the fastener has a porous mesh structure in the anchor section and is placed to promote tissue growth across the defect provides a significant advantage over all other fastening means currently practiced.

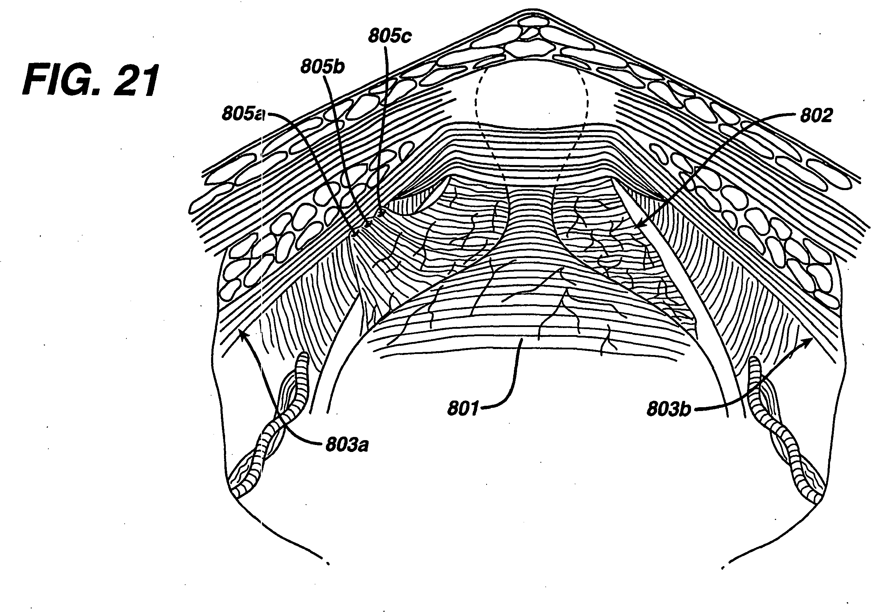

FIG. 21 is an illustration of the pelvic cavity of a female as seen from above and viewed from dorsal to ventral. This view illustrates how the fastener of the present invention is utilized to suspend the bladder 801 above the vagina, not shown, to repair a cystocele, which is the intrusion of the bladder into the vaginal space. This type prolapse is sometimes caused by a tear or rip in the endopelvic fascia 802, which connects between the fascial white lines 803a and 803b on either side of the pelvis. In this type of repair, the endopelvic fascia 802 is re-approximated to the fascial white line 803a by means of a number of fasteners 805a, 805b and 805c. The fasteners can be deployed through the endopelvic fascia and into the white line by way of different surgical and operative procedures. One such procedure would be laparoscopic with the approach through the abdominal rectus muscle and access to the surgical site from outside the vaginal. A second procedure would be an open laparotomy with again the access to the surgical site from outside the vagina. A third procedure would be a trans vaginal approach with access to the surgical site through an incision in the vaginal wall.

In FIG 21 the fasteners 805a, 805b and 805c are deployed so that the endopelvic fascia 802 is compressed firmly against the fascial white line 803a. It should be noted that in making this attachment and the deployment of the fasteners through the two layers of tissue, that the potential for making an accurate measurement of the distances between the top layer of the fascia and the top layer of the white line is difficult due to the position of the site and the access to the site, especially if the laparoscopic procedure or the transvaginal procedure are used. Additionally, those skilled in the art of making this repair will understand the criticalness of making a firm approximation of the two tissues in order to have good healing and a favorable surgical outcome. Therefore, the biasing forces generated by the central biasing section of the fastener are utilized to ensure good close approximation without precise measurement. Also, the two anchors of the fastener and the connection of the connector section to them increases the holding force of the fastener and prevents unintentional pullout from the fascial white line, or tear through of the proximal section of the fastener through the endopelvic fascia.

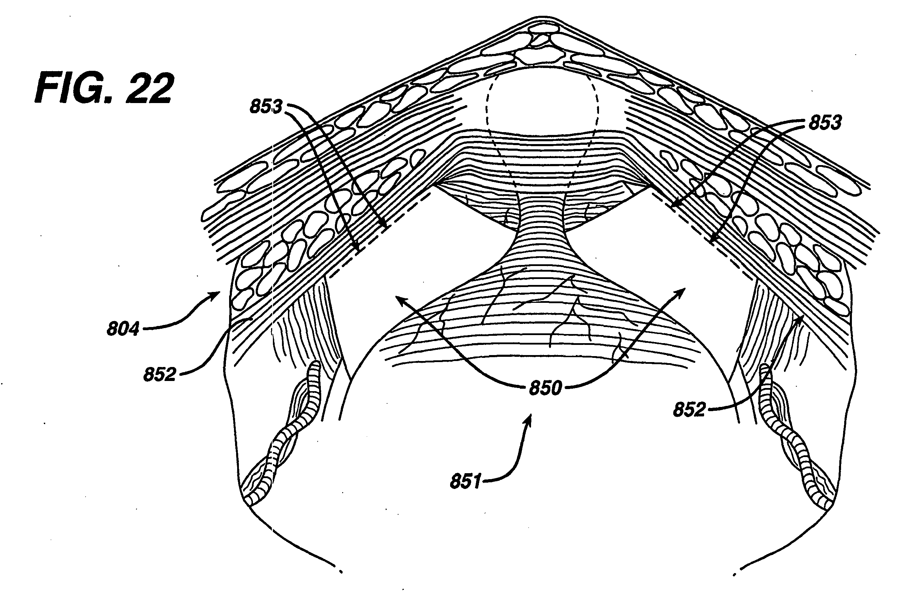

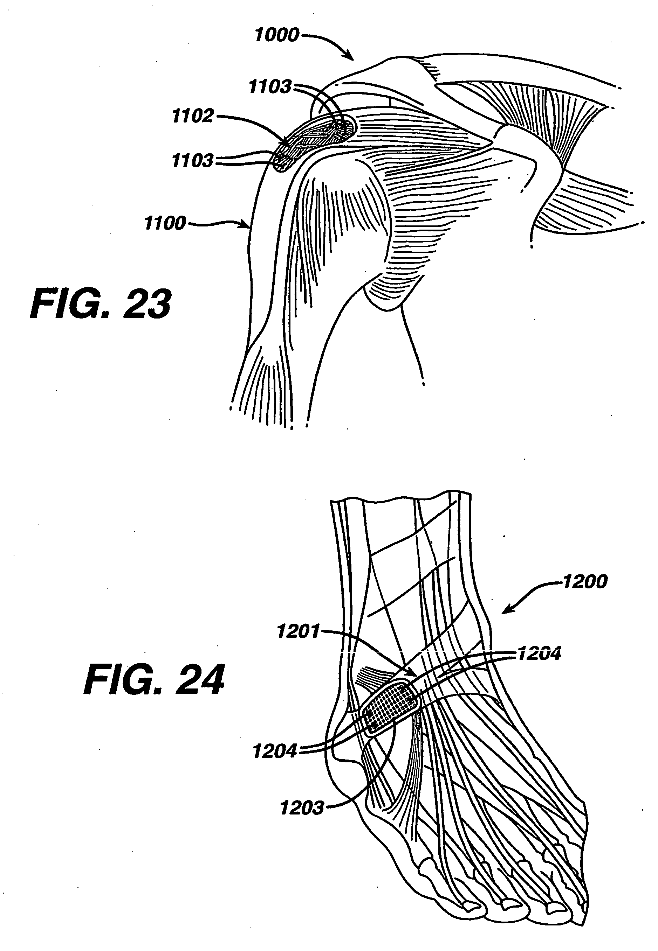

FIG. 22 illustrates a similar repair as shown in FIG. 21, except that in place of the endopelvic fascia 802 of FIG 23 a surgical fabric is used to support the bladder 851. The fabric 850 is attached to the fascial white line 852 by the deployment of several fasteners 853 through the fabric, which hold the fabric in firm approximation to the fascial white line. The wide coverage of the connector section of the fasteners extends across a number of pores in the fabric and therefore relies on numerous filaments or yarns under the connector section to hold the fabric in place, rather than a rod or plate type footprint as is the case with known H-type fasteners.

Another use for the fasteners of the present invention is to repair a tear or the like in the tendons of the shoulder. FIG. 23 illustrates the placement of a fabric patch 1102 on the supraspinatus tendon 1100 of a human shoulder 1000 to repair the tear in the tendon. The patch 1102 is held attached to the tendon 1100 by a number of fasteners 1103 of the present invention. The patch is designed to promote tissue in growth within and through the fabric and across the tear thereby repairing it.

Yet another application of the fasteners of the present invention is to repair a tear or the like in the tendons of the ankle. FIG. 24 illustrates the placement of a fabric patch 1203 on the peroneus longus tendon 1201 of a human ankle 1200 to repair a tear in the tendon. The patch 1203 is held attached to the tendon 1201 by a number of fasteners 1204 of the present invention. The patch is designed to promote tissue in growth within and through the fabric and across the tear thereby repairing it.

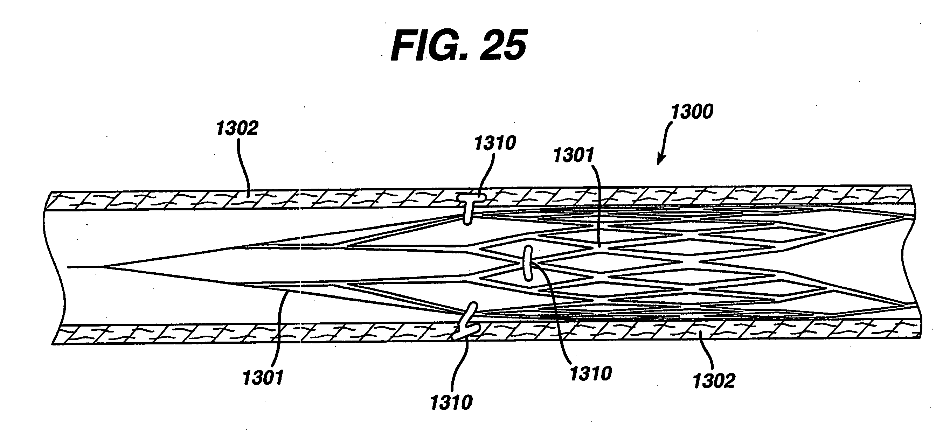

FIG. 25 illustrates the placement of a number of fasteners of the present invention within the lumen of a vessel 1300, such as an artery, vein, or other duct for carrying fluids within the human body. The fasteners 1310 are placed through a stent 1301 and into the wall 1302 of the vessel. The fasteners 1310 capture a portion of the stent 1301 between the two biasing sections of the fastener so that the connector section securely lies across a portion of the stent and attaches it to the internal wall of the vessel. The anchors of the fasteners are embedded in the wall 1302 of the vessel 1300.

Although the present invention has been shown and described with respect to detailed embodiments thereof, it will be understood by those skilled in the art that various changes in form and detail may be made without departing from the scope of the claimed invention. It will also be evident by those skilled in the art that the utility of the fastener is not limited to the surgical procedures described here. The fasteners of the present invention have application in any surgical procedure where two layers of tissue are to be approximated and the use of a fastener system is preferred.

## Claims

1. A fastener (5) for securing a target tissue to a second element comprising:
a first anchor (20a);
a second anchor (20b), the first and second anchors (20a, 20b) substantially lying in a first plane,
a connector including a first central biasing section (13) having a first end (16a) coupled to the first anchor (20a) and a second end (16b), a second central biasing section (12) having a first end (14a) coupled to the second anchor (20b) and a second end (14b), and a connector section (15) extending between the second end (16b) of the first central biasing section (13) and the second end (14b) of the second biasing section (12),
wherein the first and second central biasing sections (12, 13) are capable of being elastically deformed from a first relaxed resting position wherein the connector section (15) is positioned a first distance from the first plane, to a second extended position wherein the connector section (15) is positioned a second distance from the first plane that is greater than the first distance, and wherein when in the second extended position the connecting section is biased toward the first plane;
wherein:
the first and second anchors (20a**,** 20b) have first and second longitudinal axes (21a, 21b) respectively;
the first and second longitudinal axes (21a, 21b) are substantially parallel to and spaced apart from one another when the first and second biasing sections (12, 13) are in said resting position;
the first and second anchors (20a, 20b) are elongated cylinders each having opposed rounded ends (22);
the first and second central biasing sections (12, 13) extend from the first and second anchors respectively in between the opposed rounded ends (22) towards the connector section (15) at an acute angle relative to the anchors (20a, 20b); and
the connecting section (15) extends substantially perpendicular to the longitudinal axes (21a, 21b) of the first and second anchors (20a, 20b)

2. The fastener (5) of claim 1, wherein the first and second central biasing sections (12, 13) lie in second and third planes that are substantially parallel to one another.

3. The fastener (5) of claim 1 or claim 2, wherein the first and second central biasing sections (12, 13) each comprise at least one substantially straight section and at least one substantially curved section.

4. The fastener (5) of any one of the preceding claims, wherein the first and second central biasing sections (12, 13) each have a "D-shaped" cross- section.

5. The fastener (5) of any one of the preceding claims, wherein the fastener (5) is composed of a non-absorbable material.

6. The fastener (5) of claim 5, wherein the non-absorbable material is selected from the group consisting of polyethylene, polypropylene, nylon, polysolfoam, polyester and polyolefin.

7. The fastener (5) of any one of claims 1 to 4, wherein the fastener (5) is composed of a bioabsorbable material.

8. The fastener (5) of claim 7, wherein the bioabsorbable material is selected from the group consisting of polydioxanone, polygalactin, polylactic acid, polygalactic acid, polycaprola[sigma]tone and blends thereof.

9. The fastener(5) of any one of the preceding claims wherein the fastener (5) composed of a combination of a bioabsorbable material and a non-absorbable material.

10. The fastener (5) of any one of the preceding claims, wherein the connecting section (15) has a longitudinal axis that is substantially perpendicular to the longitudinal axes (21a, 21b) of the first and second anchors (20a, 20b).

11. The fastener (5) of any one of claims 1 to 9, wherein the connecting section (15) has as semi-circular configuration with an approximately 180° arc.

12. The fastener (5) of any one of claims 1 to 9, wherein the connecting section (15) has holes or pores (62) between solid ribs (65) that connect a top (66) of the section (15) and the bottom (67).

13. The fastener (5) of any one of the preceding claims, wherein the biasing sections (12, 13) have a saw-tooth configuration.

14. The fastener (5) of any one of claims 1 to 12, wherein the biasing sections (12, 13) have a sinusoidal configuration.

15. The fastener (5) of any one of the preceding claims, including a fabric or mesh structure in the position of the connecting section with pore sizes between 50µm and 500µm.

## Patentansprüche

1. Befestigungselement (5) zum Sichern eines Zielgewebes an einem zweiten Element, das aufweist:
einen ersten Anker (20a);
einen zweiten Anker (20b), wobei der erste und der zweite Anker (20a, 20b) im wesentlichen in einer ersten Ebene liegen,
einen Verbinder, der einen ersten mittleren vorbelastenden Abschnitt (13) mit einem ersten Ende (16a), das an den ersten Anker (20a) gekoppelt ist, und einem zweiten Ende (16b), einen zweiten mittleren vorbelastenden Abschnitt (12) mit einem ersten Ende (14a), das mit dem zweiten Anker (20b) gekoppelt ist, und einem zweiten Ende (14b) und einen Verbinderabschnitt (15), der sich zwischen dem zweiten Ende (16b) des ersten mittleren vorbelastenden Abschnittes (13) und dem zweiten Ende (14b) des zweiten vorbelastenden Abschnittes (12) erstreckt, umfaßt,
wobei der erste und der zweite mittlere vorbelastende Abschnitt (12, 13) aus einer ersten entspannten Ruheposition, in der der Verbinderabschnitt (15) einen ersten Abstand von der ersten Ebene angeordnet ist, in eine zweite aufgeweitete Position, in der der Verbinderabschnitt (15) einen zweiten Abstand von der ersten Ebene angeordnet ist, der größer als der erste Abstand ist, elastisch deformiert werden können, und wobei, wenn er in der zweiten aufgeweiteren Position ist, der Verbindungsabschnitt auf die erste Ebene zu vorbelastet ist;
wobei:
der erste und der zweite Anker (20a, 20b) eine erste bzw. eine zweite Längsachse (21a, 21 b) haben;
wobei die erste und die zweite Längsachse (21a, 21b) im wesentlichen parallel zu und beabstandet voneinander sind, wenn der erste und der zweite vorbelastende Abschnitt (12, 13) in der Ruheposition sind;
wobei der erste und der zweite Anker (20a, 20b) längliche Zylinder sind, jeder mit gegenüberliegenden gerundeten Enden (22);
wobei sich der erste und der zweite mittlere vorbelastende Abschnitt (12, 13) von dem ersten bzw. dem zweiten Anker zwischen den gegenüberliegenden gerundeten Enden (22) auf den Verbinderabschnitt (15) unter einem spitzen Winkel relativ zu den Ankern (20a, 20b) erstreckt; und
der verbindende Abschnitt (15) sich im wesentlichen senkrecht zu den Längsachsen (21 a, 21 b) des ersten und des zweiten Ankers (20a, 20b) erstreckt.

2. Befestigungselement (5) nach Anspruch 1, bei dem der erste und der zweite mittlere vorbelastende Abschnitt (12, 13) in einer zweiten und in einer dritten Ebene liegen, die im wesentlichen parallel zueinander sind.

3. Befestigungselement (5) nach Anspruch 1 oder Anspruch 2, bei dem der erste und der zweite mittlere vorbelastende Abschnitt (12, 13) jeder wenigstens einen im wesentlichen geraden Abschnitt und wenigstens einen im wesentlichen gebogenen Abschnitt aufweisen.

4. Befestigungselement (5) nach einem der vorangehenden Ansprüche, bei dem der erste und der zweite mittlere vorbelastende Abschnitt (12, 13) jeweils einen "D-förmigen" Querschnitt hat.

5. Befestigungselement (5) nach einem der vorangehenden Ansprüche, wobei das Befestigungselement (5) aus einem nicht absorbierbaren Material aufgebaut ist.

6. Befestigungselement (5) nach Anspruch 5, bei dem das nicht absorbierbare Material aus der Gruppe bestehend aus Polyethylen, Polypropylen, Nylon, Polysolschaum, Polyester und Polyolefin ausgewählt ist.

7. Befestigungselement (5) nach einem der Ansprüche 1 bis 4, wobei das Befestigungselement (5) aus einem bioabsorbierbaren Material aufgebaut ist.

8. Befestigungselement (5) nach Anspruch 7, bei dem das bioabsorbierbare Material aus der Gruppe bestehend aus Polydioxanon, Polygalactin, Polymilchsäure, Polygalactinsäure, Polycaprola[sigma]ton und Mischungen aus diesen ausgewählt ist.

9. Befestigungselement (5) nach einem der vorangehenden Ansprüche, wobei das Befestigungselement (5) aus einer Kombination aus einem bioabsorbierbaren Material und einem nicht absorbierbaren Material aufgebaut ist.

10. Befestigungselement (5) nach einem der vorangehenden Ansprüche, bei dem der verbindende Abschnitt (15) eine Längsachse hat, die im wesentlichen senkrecht zu den Längsachsen (21 a, 21 b) des ersten und des zweiten Ankers (20a, 20b) ist.

11. Befestigungselement (5) nach einem der Ansprüche 1 bis 9, bei dem der verbindende Abschnitt (15) eine halbkreisförmige Ausgestaltung mit einem Bogen von ungefähr 180° hat.

12. Befestigungselement (5) nach einem der Ansprüche 1 bis 9, bei dem der verbindende Abschnitt (15) Löcher oder Poren (62) zwischen festen Rippen (65) hat, die eine Oberseite (66) des Abschnitts (15) und die Unterseite (67) verbinden.

13. Befestigungselement (5) nach einem der vorangehenden Ansprüche, bei dem die vorbelastenden Abschnitte (12, 13) eine sägezahnartige Ausgestaltung haben.

14. Befestigungselement (5) nach einem der Ansprüche 1 bis 12, bei dem die vorbelastenden Abschnitte (12, 13) eine sinusartige Ausgestaltung haben.

15. Befestigungselement (5) nach einem der vorangehenden Ansprüche, das eine Textil- oder Netzstruktur an dem Ort des verbindenden Abschnittes mit Porengrößen zwischen 50 µm und 500 µm umfaßt.

## Revendications

1. Dispositif de fixation (5) destiné à fixer un tissu cible sur un second élément comprenant :
un premier ancrage (20a) ;
un second ancrage (20b), les premier et second ancrages (20a, 20b) étant situés sensiblement dans un premier plan,
un connecteur comportant une première section de sollicitation centrale (13) présentant une première extrémité (16a) couplée au premier ancrage (20a) et une seconde extrémité (16b), une seconde section de sollicitation centrale (12) présentant une première extrémité (14a)
couplée au second ancrage (20b) et une seconde extrémité (14b), et une section de connecteur (15) s'étendant entre la seconde extrémité (16b)
de la première section de sollicitation centrale (13) et la seconde extrémité (14b) de la seconde section de sollicitation (12),
dans lequel les première et seconde sections de sollicitation centrale (12, 13) sont capables d'être déformées élastiquement d'une première position de repos relâchée dans laquelle la section de connecteur (15) est positionnée suivant une première distance par rapport au premier plan vers une seconde position dans laquelle la section de connecteur (15) est positionnée suivant une seconde distance par rapport au premier plan qui est supérieure à la première distance, et dans lequel lorsqu'elle se trouve dans la seconde position étendue, la section de connexion est sollicitée en direction du premier plan ;
dans lequel :
les premier et second ancrages (20a, 20b) présentent des premier et second axes longitudinaux (21a, 21b) respectivement ;
les premier et second axes longitudinaux (21a, 21b) sont sensiblement parallèles l'un à l'autre et espacés l'un de l'autre lorsque les première et seconde sections de sollicitation (12, 13) se trouvent dans ladite position de repos ;
les premier et second ancrages (20a, 20b) sont des cylindres allongés présentant chacun des extrémités arrondies opposées (22) ;
les première et seconde sections de sollicitation centrale (12, 13) s'étendent des premier et second ancrages respectivement entre
les extrémités arrondies opposées (22) en
direction de la section de connecteur (15)
suivant un angle aigu par rapport aux ancrages
(20a, 20b) ; et
la section de connexion (15) s'étend
sensiblement perpendiculairement aux axes
longitudinaux (21a, 21b) des premier et second
ancrages (20a, 20b).

2. Dispositif de fixation (5) selon la revendication 1, dans lequel les première et seconde sections de sollicitation centrale (12, 13) se situent dans des deuxième et troisième plans qui sont sensiblement parallèles l'un à l'autre.

3. Dispositif de fixation (5) selon la revendication 1 ou la revendication 2, dans lequel les première et seconde sections de sollicitation centrale (12, 13) comprennent chacune au moins une section sensiblement rectiligne et au moins une section sensiblement incurvée.

4. Dispositif de fixation (5) selon l'une quelconque des revendications précédentes, dans lequel les première et seconde sections de sollicitation centrale (12, 13) présentent chacune une section transversale "en forme de D".

5. Dispositif de fixation (5) selon l'une quelconque des revendications précédentes, dans lequel le dispositif de fixation (5) est composé d'un matériau non résorbable.

6. Dispositif de fixation (5) selon la revendication 5, dans lequel le matériau non résorbable est sélectionné à partir du groupe constitué de polyéthylène, polypropylène, nylon, mousse polysol, polyester et polyoléfine.

7. Dispositif de fixation (5) selon l'une quelconque des revendications 1 à 4, dans lequel le dispositif de fixation (5) est composé d'un matériau biorésorbable.

8. Dispositif de fixation (5) selon la revendication 7, dans lequel le matériau biorésorbable est sélectionné parmi le groupe constitué de polydioxanone, polygalactine, acide polylactique, acide polygalactique, polycaprola[sigma]tone et ses mélanges.

9. Dispositif de fixation (5) selon l'une quelconque des revendications précédentes dans lequel le dispositif de fixation (5) est composé d'une combinaison d'un matériau biorésorbable et d'un matériau non résorbable.

10. Dispositif de fixation (5) selon l'une quelconque des revendications précédentes, dans lequel la section de connexion (15) présente un axe longitudinal qui est sensiblement perpendiculaire aux axes longitudinaux (21a, 21b) des premier et second ancrages (20a, 20b).

11. Dispositif de fixation (5) selon l'une quelconque des revendications 1 à 9, dans lequel la section de connexion (15) présente une configuration semi-circulaire dotée d'un arc d'approximativement 180°.

12. Dispositif de fixation (5) selon l'une quelconque des revendications 1 à 9, dans lequel la section de connexion (15) présente des trous ou des portes (62) entre des nervures solides (65) qui raccordent une partie supérieure (66) de la section (15) et la partie inférieure (67).

13. Dispositif de fixation (5) selon l'une quelconque des revendications précédentes, dans lequel les sections de sollicitation (12, 13) présentent une configuration en dent de scie.

14. Dispositif de fixation (5) selon l'une quelconque des revendications 1 à 12, dans lequel les sections de sollicitations (12, 13) présentent une configuration sinusoïdale.

15. Dispositif de fixation (5) selon l'une quelconque des revendications précédentes, comportant une structure de tissu ou de maillage dans la position de la section de raccordement avec des tailles de pore comprises entre 50 µm et 500 µm.
